# EUROPÄISCHE PATENTSCHRIFT

(11) **EP 3 334 476 B1**
(45) Veröffentlichungstag und Bekanntmachung des Hinweises auf die Patenterteilung: **28.09.2022**
(21) Anmeldenummer: 16754190.3
(22) Anmeldetag: 10.08.2016
(51) Int. Cl.: A61M 1/28, G01G 23/06, G01G 23/08, G01G 23/10, G01G 23/12, A61M 1/16, G01G 23/00

(54) **PERITONEALDIALYSEGERÄT**
PERITONEAL DIALYSIS DEVICE
APPAREIL DE DIALYSE PÉRITONÉALE

(30) Priorität: 11.08.2015 DE 102015010468
(43) Veröffentlichungstag der Anmeldung: 20.06.2018
(73) Patentinhaber: Fresenius Medical Care Deutschland GmbH, 61352 Bad Homburg (DE)
(72) Erfinder: ZEYHER, Peter, 64289 Darmstadt (DE); FRITSCH, Kim, 65468 Trebur (DE)
(74) Vertreter: Herrmann, Uwe
(86) Internationale Anmeldenummer: PCT/EP2016/001378
(87) Internationale Veröffentlichungsnummer: WO 2017/025196

(56) Entgegenhaltungen:
- WO-A1-84/02277
- US-A- 4 479 561
- US-A- 4 726 435
- US-A1- 2009 187 138
- US-A1- 2011 160 649

## Beschreibung

Die vorliegende Erfindung betrifft ein Peritonealdialysegerät mit wenigstens einem Gerätegehäuse und mit wenigstens einer Drainschale zur Aufnahme eines oder mehrerer Lösungsbeutel zur Aufbewahrung von gebrauchtem Dialysat, wobei die Drainschale mit wenigstens einer Wägezelle derart in Verbindung steht, dass das Gewicht der Drainschale mittels der Wägezelle erfassbar ist.

Bei aus dem Stand der Technik bekannten Peritonealdialysegeräten befindet sich unterhalb des Gerätegehäuses eine Schale, im Folgenden auch als Drainschale bezeichnet, die zur Aufnahme eines oder mehrerer Beutel dient, die mit Dialysat gefüllt sind bzw. gefüllt werden, das aus den Patienten abgeführt wird. Diese Schale steht über einen Verbindungsmechanismus, wie z.B. ein Gestänge mit einer Wägezelle in Verbindung, die sich üblicherweise in oder an dem Gerätegehäuse befindet. Die Wägezelle ermittelt das Gewicht der Drainschale bzw. des oder der darin befindlichen Beutel.

Bei unzulässiger Krafteinwirkung bzw. ungleichmäßiger Lastenverteilung in der Drainschale, kann ein Zustand auftreten, der die Wägezelle überlastet bzw. beschädigt. Eine solche unzulässige Belastung bzw. Krafteinwirkung kann beispielsweise dann eintreten, wenn der Nutzer des Gerätes versehentlich die Drainschale über ein bestimmtes Maß hinaus auslenkt oder beispielsweise auch dann, wenn die Drainschale außerhalb des Schwerpunktes mit Lösungsbeuteln belastet wird. Auch Schwingungen der Drainschale können zu einer Beschädigung bzw. Überlastung der Wägezelle führen.

Die US 2011 / 160 649 A1 und die US 44 726 435 A offenbaren ein Peritonealdialysegerät gemäß dem Oberbegriff des Anspruchs 1.

Der vorliegenden Erfindung liegt daher die Aufgabe zugrunde, ein Peritonealdialysegerät der eingangs genannten Art dahingehend weiterzubilden, dass eine Überlastung der Wägezelle auf vergleichsweise einfache Art und Weise zuverlässig verhindert wird.

Diese Aufgabe wird durch ein Peritonealdialysegerät der eingangs genannten Art dadurch gelöst, dass zumindest ein Dämpfungssystem vorgesehen ist, das das Auftreten unzulässiger Krafteinleitung in die Wägezelle verhindert. Das Dämpfungssystem weist wenigstens einen Dämpfer auf, an dem die Drainschale unmittelbar oder mittelbar über eine Halterung befestigt ist, wobei der Dämpfer mit der Wägezelle so in Verbindung steht, dass das auf diesen wirkende Gewicht der Drainschale auf die Wägezelle übertragen wird.

Die Erfindung wird durch die Merkmale des unabhängigen Anspruchs 1 definiert. Erfindungsgemäß ist somit vorgesehen, dass die Drainschale bzw. deren Halterung, wie beispielsweise ein Gestänge oder dergleichen nicht unmittelbar mit der Wägezelle in Verbindung steht, sondern mittelbar über wenigstens einen Dämpfer. Dieser Dämpfer schützt die Wägezelle nicht nur vor unzulässiger Krafteinwirkung bzw. den Folgen ungleichmäßiger Lastverteilung, sondern dämpft auch Schwingungen, in die die Drainschale versehentlich versetzt wurde. Die Belastung der Wägezelle wird somit entsprechend verringert, da die Bewegungen bzw. die Kräfte und Momente, die auf die Drainschale einwirken, nicht unmittelbar, sondern nur mittelbar und gedämpft an die Wägezelle übertragen werden.

Das Dämpfungssystem kann somit als Kraft- und Drehmomentbegrenzungseinrichtung fungieren, die das auf die Wägezelle wirkende Drehmoment bzw. die auf die Wägezelle wirkende Kraft - verursacht durch die Drainschale bzw. deren Halterung - dämpft bzw. verringert.

An dieser Stelle wird darauf hingewiesen, dass der Begriff "Gewicht der Drainschale" sowohl das Gewicht der leeren Drainschale als auch das Gewicht der mit einem oder mehreren Lösungsbeuteln befüllten Drainschale umfasst.

Des Weiteren wird darauf hingewiesen, dass der Begriff "Lösungsbeutel" stellvertretend für jedes Behältnis mit starren oder flexiblen Wandungen steht, das zur Aufnahme gebrauchten Dialysats geeignet ist. Der Begriff ist somit nicht auf einem Beutel im engeren Sinne beschränkt.

Schließlich wird darauf hingewiesen, dass der Begriff "Drainschale" jede beliebige Aufnahme, z.B. für einen oder mehrere Beutel oder Behälter mit flexiblen oder nicht flexiblen Wandungen umfassen kann, als auch stellvertretend für jedes beliebige Gefäß stehen kann, in das eine Flüssigkeit, vorzugsweise verbrauchte Dialyselösung aufgenommen werden kann. Bei der "Drainschale" kann es sich somit beispielsweise auch unmittelbar um einen Container oder Beutel handeln, der mit der Wägezelle in Verbindung steht und dessen Gewicht gemessen wird - unabhängig davon, ob eine Schale zur Aufnahme des Containers oder Beutels verwendet wird oder nicht.

Des Weiteren wird darauf hingewiesen, dass die Wägezelle vorzugsweise in einem Gehäuse der Wägezelle angeordnet ist oder einen Bestandteil des Gehäuses der Wägezelle bildet.

In einer bevorzugten Ausgestaltung der Erfindung ist das Dämpfungssystem des Weiteren so ausgeführt, dass die Drainschale bzw. deren Halterung nicht mit einem oder mehreren weiteren Elementen des Peritonealdialysegerätes in Verbindung kommt, was eine Verfälschung des Messergebnisses zur Folge hätte.

Erfindungsgemäß ist vorgesehen, dass die Drainschale nicht unmittelbar, sondern mittelbar über wenigstens eine Halterung und ggf. Bestandteile des Gehäuses der Wägezelle an der Wägezelle angeordnet ist.

Erfindungsgemäß ist vorgesehen, dass der Dämpfer zumindest eine Durchführung aufweist, durch die sich die Halterung hindurch erstreckt. Diese Halterung kann im Querschnitt kreisförmig ausgebildet sein, jedoch ist auch jede beliebige andere Durchführung denkbar und von der Erfindung mit umfasst.

Vorzugsweise erstreckt sich die Durchführung in senkrechter Richtung durch den Dämpfer.

Der Dämpfer kann eine Längsachse aufweisen und die Durchführung erstreckt sich vorzugsweise entlang oder parallel zu der Längsachse.

Vorzugsweise befindet sich die Durchführung im Zentrum des Dämpfers bzw. verläuft durch dessen mittigen Bereich.

In einer weiteren Ausgestaltung der Erfindung ist vorgesehen, dass die Dämpfungseinrichtung zumindest ein Kraftübertragungselement, vorzugsweise wenigstens eine Bodenplatte aufweist, die sich zwischen dem Dämpfer und dem Gehäuse der Wägezelle erstreckt. In dieser Ausführungsform der Erfindung steht der Dämpfer somit nicht unmittelbar mit dem Gehäuse der Wägezelle in Verbindung. Vielmehr ist zwischen dem Dämpfer und dem Gehäuse der Wägezelle wenigstens ein Kraftübertragungselement, z.B. aus Metall oder Kunststoff angeordnet, das die auf den Dämpfer wirkende Kraft auf die Wägezelle bzw. auf deren Gehäuse überträgt.

Dabei kann es sich um eine Bodenplatte handeln, die zwischen dem Dämpfer und dem Gehäuse der Wägezelle angeordnet ist. Denkbar ist es, dass die Bodenplatte zwischen dem Dämpfer und der den Boden des Gehäuses bildenden Montageplatte des Gehäuses der Wägezelle angeordnet ist. Grundsätzlich kommt auch jedes andere Element in Frage, das für eine solche Kraftübertragung geeignet ist. Das Kraftübertragungselement muss daher nicht zwingend plattenförmig ausgeführt sein.

Erfindungsgemäß ist vorgesehen, dass die Dämpfungseinrichtung zumindest ein Krafteinleitungselement aufweist, mittels derer die durch die Drainschale bzw. die darin befindlichen Beutel aufgebrachte Kraft in den Dämpfer eingeleitet wird. Dieses Krafteinleitungselement ist in Form einer Deckplatte ausgebildet, die oben auf den Dämpfer drückt bzw. die oberhalb des Dämpfers angeordnet ist. Auch das Krafteinleitungselement muss nicht zwingend plattenförmig ausgeführt sein, auch jede andere Form des Krafteinleitungselementes ist denkbar und von der Erfindung mit umfasst.

Das Krafteinleitungselement kann beispielsweise aus Metall oder Kunststoff bestehen.

In einer bevorzugten Ausgestaltung der Erfindung ist vorgesehen, dass das Krafteinleitungselement, wie beispielsweise die Deckplatte und/oder das Kraftübertragungselement, wie beispielsweise die Bodenplatte eine Größe aufweist, die der Fläche des Dämpfers entspricht, die mit dem Krafteinleitungselement bzw. mit dem Kraftübertragungselement in Verbindung steht. Durch eine solche vollflächige Kontaktierung wird erreicht, dass die jeweils eingeleiteten bzw. zu übertragenden Kräfte über eine relativ große Fläche des Dämpfers übertragen werden und somit die Dämpfungseigenschaften optimal ausgenutzt werden.

Der Dämpfer kann zylindrisch ausgeführt sein, jedoch auch jede beliebige andere Form aufweisen.

Vorzugsweise besteht der Dämpfer aus Gummi oder weist zumindest Gummi auf. Auch andere dämpfende bzw. elastische Materialien sind denkbar und von der Verbindung mit umfasst.

Die Drainschale ist an wenigstens einer Halterung, wie beispielsweise an einem Gestänge oder dergleichen angeordnet, die sich abschnittsweise durch eine Öffnung des Gehäuses erstreckt, in oder an dem sich die Wägezelle befindet, wobei die Öffnung so dimensioniert ist, dass die Halterung in wenigstens einer Position der Drainschale nicht den Öffnungsrand der Öffnung des Gehäuses der Wägezelle berührt. Dadurch wird sichergestellt, dass eine Verfälschung des Messergebnisses durch Kontakt der Halterung mit einem Teil des Peritonealdialysegerätes verhindert wird. Vorzugsweise ist vorgesehen, dass es sich bei der genannten Position um die Position der Drainschale handelt, in der sich diese in ihrer nicht belasteten Ruheposition befindet.

Denkbar ist es, dass die genannte Öffnung einen Durchmesser aufweist, der zumindest doppelt so groß ist wie der Außendurchmesser der Halterung. Somit ist eine gewisse Auslenkung der Halterung ausgehend von der Ruheposition denkbar, wobei auch in der ausgelenkten Position eine zuverlässige Gewichtsermittlung durch die Wägezelle möglich ist.

Vorzugsweise befindet sich die Wägezelle in oder an dem Gerätegehäuse und die Drainschale unterhalb des Gerätegehäuses.

Bevorzugt ist es, wenn sich die Wägezelle im Gerätegehäuse befindet und die Drainschale unterhalb des Gerätegehäuses.

Um die Verformungswerte der Wägezelle bzw. des Gehäuses der Wägezelle beschränken zu können, ist vorzugsweise wenigstens ein Anschlag vorgesehen, der diese Verformung begrenzt.

In einer bevorzugten Ausgestaltung der Erfindung befindet sich dieser Anschlag unterhalb des Gehäuses der Wägezelle. Der Anschlag kann beispielsweise als Platte, insbesondere als Metallplatte ausgeführt sein, die sich unterhalb der Montageplatte des Gehäuses der Wägezelle erstreckt und von dieser wenigstens bereichsweise beabstandet ist. In Betracht kommt beispielsweise eine Stahl- oder Aluminiumplatte.

Um die Auslenkung begrenzen und den Eintrag einer unzulässig hohen dynamischen oder statischen Krafteinwirkung auf die Wägezelle sicher abfangen zu können, ist daher vorzugsweise ein derartiger Anschlag vorgesehen.

Vorteilhaft ist es, wenn dieser Anschlag verstellbar ist. So können Verstellmittel vorgesehen sein, mittels derer der Abstand zwischen dem Gehäuse der Wägezelle und dem Anschlag veränderbar ist, wobei vorzugsweise vorgesehen ist, dass die Verstellmittel wenigstens eine Schraube und wenigstens eine Sicherungsmutter umfassen. Denkbar ist es beispielsweise, wenn in einen einstellbaren Spalt zwischen dem Montageblock, d.h. dem Gehäuse der Wägezelle und der genannten Platte eine Schraube mit einem Feingewinde und einer Verstell- und/oder Sicherungsmutter eingesetzt wird und die exakte Justierung über die Schraube vorgenommen wird.

Weitere Einzelheiten und Vorteile der Erfindung werden anhand eines in der Zeichnung dargestellten Ausführungsbeispiels näher erläutert.

Es zeigen:
- Figur 1:: eine perspektivische Ansicht eines Peritonealdialysegerätes gemäß der Erfindung,
- Figur 2:: eine perspektivische Schnittansicht durch die Dämpfungsvorrichtung mit Wägezelle gemäß der Erfindung, und
- Figur 3:: eine schematische Schnittansicht der Anordnung gemäß Figur 2-und
- Figur 4:: eine schematische Ansicht des Gehäuses der Wägezelle mit verstellbarem Anschlag.

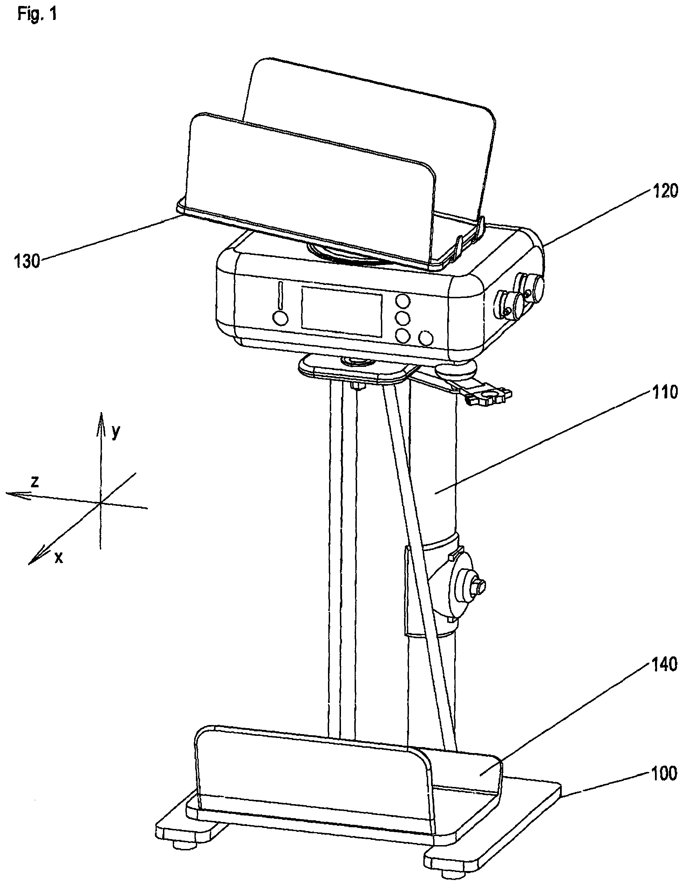

Aus Figur 1 ist ein Peritonealdialysegerät gemäß der Erfindung ersichtlich. Das Gerät weist einen Gerätefuß 100, einen sich von diesem nach oben erstreckenden Gehäuseträger 110 und ein Gerätegehäuse 120 auf, das an den Gehäuseträger 110 angeordnet ist.

In dem Gerätegehäuse 120 befindet sich die für den Betrieb des Gerätes notwendige Steuerung und ggf. Anzeige- und/oder Bedienelemente.

Oberhalb des Gerätegehäuses 120 befindet sich die Wägeschale 130. Diese ist beheizbar und dient zur Aufnahme von Lösungsbeuteln, die frisches, dem Patienten zuzuführendes Dialysat beinhalten.

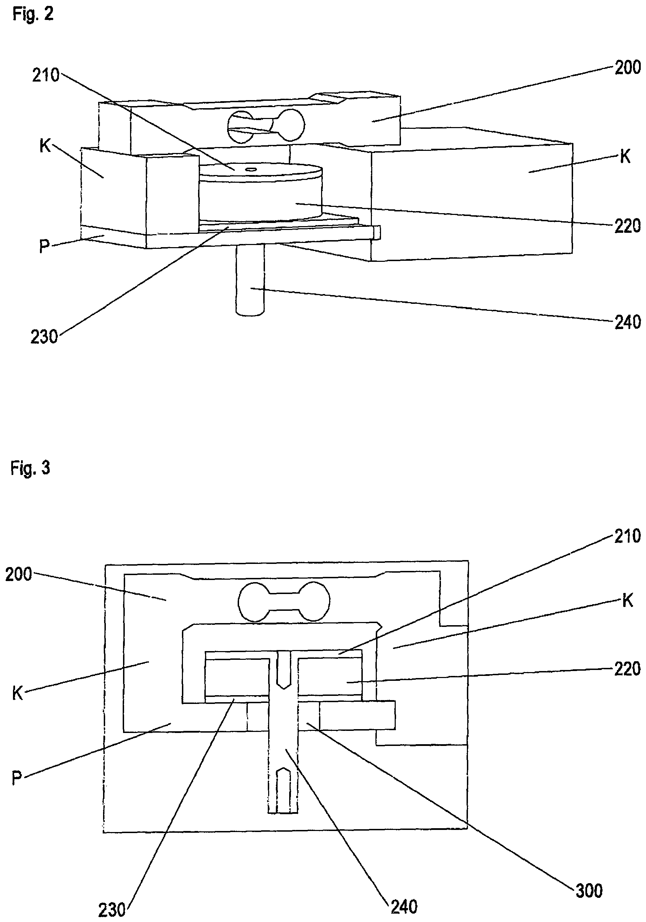

In dem Gerätegehäuse 120 ist eine Wägezelle angeordnet, die aus Figur 2 und 3 ersichtlich ist und dort mit dem Bezugszeichen 200 gekennzeichnet ist.

Von dieser Wägezelle bzw. von deren Gehäuse nach unten erstreckt sich eine Halterung, die gemäß Figur 1 in Form zweier Stangen ausgeführt ist und an denen die Drainschale 140 angeordnet ist.

Gemäß dem in Figur 1 dargestellten Ausführungsbeispiel erstreckt sich die x-Achse aus Sicht eines vor dem Gerät befindlichen Nutzers in Tiefenrichtung der Drainschale 140, die z-Achse in Breitenrichtung der Drainschale 140 und die y-Achse nach oben, das heißt parallel zu dem Gehäuseträger 110.

Eine Belastung bzw. ein Drehmoment um die x-Achse oder um die z-Achse kann beispielsweise bei einer unbeabsichtigten Berührung der Drainschale 140 auftreten.

Ein Drehmoment um die y-Achse kann beispielsweise auftreten, wenn jemand die Drainschale um diese Achse dreht.

Die Dämpfungsvorrichtung gemäß der vorliegenden Erfindung dient dazu, die durch die Drainschale bedingten Belastungen der Wägezelle zu dämpfen bzw. zu verringern.

Figur 2 zeigt mit dem Bezugszeichen 200 die Wägezelle, die sich innerhalb des Gerätegehäuses 120 des Peritonealdialysegerätes befindet.

Die Wägezelle 200 ist in oder an einem Wägezellengehäuse angeordnet, das eine unten liegende Montageplatte P und zwei sich davon ausgehend nach oben erstreckende Montageklötze K umfasst. Die Montageplatte P befindet sich unten, unterhalb der Bodenplatte 230. Von der Montageplatte P erstrecken sich zwei Montageklötze K rechts und links von dem Dämpfer 220 nach oben. Die Montageklötze K stehen an ihrer Oberseite mit der Wägezelle 200 in Verbindung.

Die Montageplatte P, die beiden Montageklötze K und die Wägezelle 200 umgeben eine Ausnehmung, in der sich die Dämpfungsvorrichtung befindet.

Die Dämpfungsvorrichtung umfasst eine z.B. kreisförmige Deckplatte 210, eine Bodenplatte 230 und den Dämpfer 220, der zwischen den Platten 210 und 230 angeordnet ist. Die Deckplatte 210 hat dieselbe Dimension wie der Dämpfer 220, sodass diese bündig mit dem Dämpfer abschließt. Die Bodenplatte 230 kann ebenfalls diese Form aufweisen oder auch rechteckig oder quadratisch ausgeführt sein. Die Bodenplatte 230 ist vorzugsweise größer ausgeführt als der Dämpfer 200, so dass diese an einer oder an mehreren Seiten entsprechend übersteht.

Über die Bodenplatte 230 und das Wägezellengehäuse wird die auf den Dämpfer 220 bzw. auf die Deckplatte 210 wirkende Kraft bzw. das wirkende Moment an die Wägezelle 200 übertragen.

Das Bezugszeichen 240 kennzeichnet den oberen Abschnitt einer Halterung bzw. eines Gestänges, an dem die Drainschale mittelbar oder unmittelbar befestigt ist.

Den Aufbau der Anordnung gemäß Figur 2 zeigt nochmals Figur 3 in einer schematischen Schnittdarstellung.

In dieser Figur sind die gleichen bzw. funktionsgleiche Bauteile mit denselben Bezugszeichen gekennzeichnet wie in Figur 2.

Aus Figur 3 ist ersichtlich, dass der Dämpfer 220 zwischen den Platten 210 und 230 angeordnet ist.

Das Gehäuse der Wägezelle 200 bzw. die Montageplatte P weist eine Bohrung bzw. Öffnung 300 auf, durch die sich die Halterung bzw. Stange 240 hindurch erstreckt. Auch die Bodenplatte 230 weist eine solche Ausnehmung auf. Die Öffnung der Bodenplatte 230 kann genauso groß ausgeführt sein, wie die der Montageplatte P.

Des Weiteren weist der Dämpfer 220 eine zentrale und vertikal verlaufende Durchführung auf, die sich durch die Längsachse des Dämpfers 220 hindurch erstreckt.

Durch diese Durchführung des Dämpfers 220 sowie durch die Bohrung 300 bzw. Öffnung der Bodenplatte 230 sowie der Montageplatte P erstreckt sich die Stange 240.

An der Stange 240 ist die Drainschale 140 befestigt. Das Gewicht der Drainschale wird somit über die Stange 240 an die Platte 210 und von dieser auf den Dämpfer 220 übertragen. Der Dämpfer 220 überträgt die Gewichtskraft über die Platte 230 an das Gehäuse der Wägezelle 200 und somit an die Wägezelle 200. Dies gilt für die Übertragung von Momenten entsprechend.

Um zu verhindern, dass die Halterung 240 an das Gehäuse der Wägezelle 200 anschlägt, ist vorgesehen, dass die Öffnung 300 der Montageplatte P sowie auch die Öffnung der Bodenplatte 230 wesentlich größer ausgeführt ist, als die äußeren Abmessungen der Halterung 240. Somit ist auch eine gewisse Auslenkung der Halterung 240 relativ zu der in Figur 3 dargestellten Position denkbar, ohne dass eine Verfälschung der Messung durch Kontaktierung mit einem Bestandteil des Gehäuses der Wägezelle 200 oder eines sonstigen Teils stattfindet. Somit kann auch im Falle einer ungleichmäßigen Belastung der Drainschale 140 eine zuverlässige Gewichtsmessung vorgenommen werden.

Der Dämpfer 220 limitiert das auf die Wägezelle 200 wirkende Drehmoment bzw. verhindert unzulässige Krafteinwirkungen auf die Wägezelle 200. Er hat des Weiteren die Aufgabe, Schwingungen der Halterung 240 bzw. der Drainschale 140 zu dämpfen.

Der Dämpfer 220 besteht aus einem elastischen Material. Vorzugsweise besteht er aus Gummi, wobei die Erfindung nicht auf Gummi als Dämpfungsmaterial beschränkt ist.

Der Dämpfer 220 ist so ausgelegt, dass er der Schwenkbewegung der Halterung 240 ein so großes Gegenmoment entgegenbringt, dass die Drainschale bzw. die Halterung nicht weitere Bestandteile des Peritonealdialysegerätes berührt.

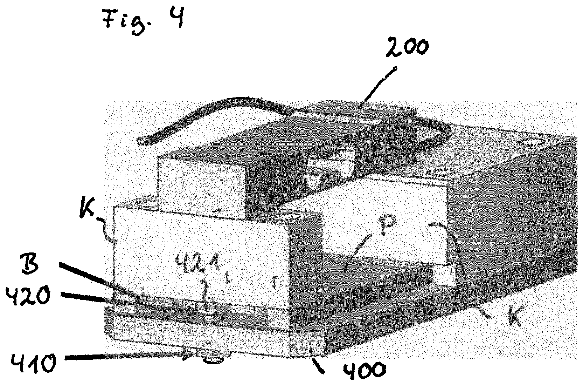

Figur 4 zeigt mit dem Bezugszeichen 200 erneut die Wägezelle sowie die Montageklötze K sowie die Montageplatte P, die zusammen das Gehäuse der Wägezelle bilden.

Wie dies weiter aus Figur 4 hervorgeht, befindet sich unterhalb der Montageplatte P eine weitere Platte 400, die von der Montageplatte P durch einen Spalt beabstandet ist.

Diese Platte 400 kann beispielsweise eine Dicke von 10 mm im Falle einer Aluminiumplatte oder 6 mm im Falle eine Stahlplatte aufweisen. Dabei handelt es sich nur um exemplarische Werte. Vorzugsweise liegt die Dicke der Platte 400 in der Spanne von 4 mm bis 12 mm. Die Platte ist vorzugsweise ausgelegt, um eine Last von bis zu 80 kg tragen zu können.

Bei einer Überlast schlägt die Montageplatte P an die Platte 400 an bzw. liegt an dieser an, so dass die Kraft von der Platte 400 aufgenommen wird.

Das Bezugszeichen B kennzeichnet eine Aussparung der Montageplatte P, in der sich eine Schraube 420 mit einem Feingewinde und eine Verstellmutter 421 befinden, durch die die Schraube 420 verdrehbar ist. Die Verstellmutter 421 ist drehfest bzw. einteilig mit der Schraube 420 verbunden. Durch das Drehen der Schraube 420 kann der Abstand zwischen der Montageplatte P und der Platte 400, d.h. dem Anschlag exakt verändert werden.

Ist die gewünschte Beabstandung erreicht, wird die Schraube 420 und damit auch die Größe des Spaltes zwischen der Montageplatte P und der Platte 400 durch die Sicherungsmutter 410 fixiert, die sich unterhalb der Platte 400 befindet und die Schraube 420 dort fixiert.

Die genannte Einstellung des Spaltmaßes kann erfolgen, bevor die Einheit gemäß Figur 4 in das Gerät eingesetzt ist. Durch die seitliche Anordnung der Verstellmutter 421 ist diese gut erreichbar, so dass die Anschlagsjustierung vergleichsweise einfach vorgenommen werden kann.

## Patentansprüche

1. Peritonealdialysegerät mit wenigstens einem Gerätegehäuse (120) und mit wenigstens einer Drainschale (140) zur Aufnahme eines oder mehrerer Lösungsbeutel zur Aufbewahrung von gebrauchtem Dialysat, wobei die Drainschale (140) mit wenigstens einer Wägezelle (200) derart in Verbindung steht, dass das Gewicht der Drainschale (140) mittels der Wägezelle (200) erfassbar ist,
**dadurch gekennzeichnet,**
**dass** wenigstens ein Dämpfungssystem vorgesehen ist, um das Auftreten unzulässiger Krafteinleitung in die Wägezelle (200) zu verhindern, wobei das Dämpfungssystem wenigstens einen Dämpfer (220) aufweist, an dem die Drainschale (140) unmittelbar oder mittelbar befestigt ist, wobei der Dämpfer (220) mit der Wägezelle (200) derart in Verbindung steht, dass das auf diesen wirkende Gewicht der Drainschale (140) auf die Wägezelle (200) übertragen wird, wobei wenigstens eine Halterung (240) vorgesehen ist, die die Drainschale (140) trägt und die unmittelbar oder mittelbar an dem Dämpfer (220) angeordnet ist und der Dämpfer (220) wenigstens eine Durchführung aufweist, durch die sich die Halterung (240) hindurch erstreckt und wobei die Dämpfungseinrichtung wenigstens ein Krafteinleitungselement in Form einer Deckplatte (210) aufweist, die sich oberhalb des Dämpfers (220) befindet und an der die Drainschale (140) mittels der Halterung (240) befestigt ist.

2. Peritonealdialysegerät nach Anspruch 1, **dadurch gekennzeichnet, dass** der Dämpfer (220) eine Längsachse aufweist und dass sich die Durchführung entlang der Längsachse oder entlang einer dazu parallel verlaufenden Achse erstreckt.

3. Peritonealdialysegerät nach einem der vorhergehenden Ansprüche, **dadurch gekennzeichnet, dass** die Dämpfungseinrichtung wenigstens ein Kraftübertragungselement, vorzugsweise wenigstens eine Bodenplatte (230) aufweist, die zwischen dem Dämpfer (220) und dem Gehäuse der Wägezelle (200) angeordnet ist.

4. Peritonealdialysegerät nach Anspruch 1 oder 3, **dadurch gekennzeichnet, dass** das Krafteinleitungselement, vorzugsweise die Bodenplatte (230) und/oder das Kraftübertragungselement, vorzugsweise die Deckplatte (210) eine Größe aufweist, die der Fläche des Dämpfers (220) entspricht, die mit dem Krafteinleitungselement und/oder mit dem Kraftübertragungselement in Verbindung steht.

5. Peritonealdialysegerät nach einem der vorhergehenden Ansprüche, **dadurch gekennzeichnet, dass** der Dämpfer (220) zylindrisch ausgeführt ist.

6. Peritonealdialysegerät nach einem der vorhergehenden Ansprüche, **dadurch gekennzeichnet, dass** der Dämpfer (220) aus Gummi besteht oder Gummi aufweist.

7. Peritonealdialysegerät nach einem der Ansprüche 2 bis 6, **dadurch gekennzeichnet, dass** sich die Halterung (240) durch wenigstens eine Öffnung (300) des Gehäuses der Wägezelle (200) erstreckt, wobei die Öffnung derart dimensioniert ist, dass die Halterung (240) in wenigstens einer Position der Drainschale (140) nicht den Öffnungsrand berührt.

8. Peritonealdialysegerät nach Anspruch 7, **dadurch gekennzeichnet, dass** es sich bei der genannten Position um die Position der Drainschale (140) handelt, in der sich diese in ihrer nicht belasteten Ruheposition befindet, wobei vorzugsweise vorgesehen ist, dass die Öffnung (300) einen Durchmesser aufweist, der zumindest doppelt so groß ist, wie der Außendurchmesser der Halterung (240).

9. Peritonealdialysegerät nach einem der vorhergehenden Ansprüche, **dadurch gekennzeichnet, dass** die Wägezelle (200) in oder an dem Gerätegehäuse (120) angeordnet ist.

10. Peritonealdialysegerät nach einem der vorhergehenden Ansprüche, **dadurch gekennzeichnet, dass** sich die Drainschale (140) unterhalb des Gerätegehäuses (120) und die Wägezelle (200) in dem Gerätegehäuse (120) befinden.

11. Peritonaldialysegerät nach einem der vorhergehenden Ansprüche, **dadurch gekennzeichnet, dass** sich unterhalb des Gehäuses der Wägezelle (200) wenigstens ein vorzugsweise verstellbarer Anschlag (400) befindet, der die Auslenkung des Gehäuses der Wägezelle (200) begrenzt.

12. Perionealdialysegerät nach Anspruch 11, **dadurch gekennzeichnet, dass** der Anschlag als Platte (400), insbesondere als Metallplatte ausgeführt ist, die sich unterhalb der Montageplatte (P) erstreckt und von dieser wenigstens bereichsweise beabstandet ist.

13. Peritonealdialysegerät nach Anspruch 11 oder 12, **dadurch gekennzeichnet, dass** Verstellmittel (420, 421) vorgesehen sind, mittels derer der Abstand zwischen dem Gehäuse der Wägezelle (200) und dem Anschlag (400) veränderbar ist, wobei vorzugsweise vorgesehen ist, dass die Verstellmittel (420, 421) wenigstens eine Schraube (420) und wenigstens eine Sicherungsmutter (421) umfassen.

## Claims

1. A peritoneal dialysis machine having at least one machine housing (120) and having at least one drain pan (140) for receiving one or more solution bags for storing consumed dialyzate, wherein the drain pan (140) is connected to at least one weighing cell (200) such that the weight of the drain pan (140) can be detected by means of the weighing cell (200),
**characterized in that**
at least one damping system is provided to prevent the occurrence of undue force introduction into the weighing cell (200), wherein the damping system has at least one damper (220) to which the drain pan (140) is directly or indirectly fastened, wherein the damper (220) is connected to the weighing cell (200) such that the weight of the drain pan (140) acting thereon is transmitted to the weighing cell (200), wherein at least one holder (240) is provided which carries the drain pan (140) and which is arranged directly or indirectly at the damper (220) and the damper (220) has at least one feed-through through which the holder (240) extends, and wherein the damping device has at least one force introduction element in the form of a top plate (210), which is located above the damper (220) and to which the drain pan (140) is fastened by means of the holder (240).

2. A peritoneal dialysis machine in accordance with claim 1, **characterized in that** the damper (220) has a longitudinal axis; and **in that** the feed-through extends along the longitudinal axis or along an axis extending in parallel therewith.

3. A peritoneal dialysis machine in accordance with one of the preceding claims, **characterized in that** the damping device has at least one force transmission element, preferably at least one base plate (230), which is arranged between the damper (220) and the housing of the weighing cell (200).

4. A peritoneal dialysis machine in accordance with claim 1 or claim 3, **characterized in that** the force introduction element, preferably the base plate (230), and/or the force transmission element, preferably the top plate (210), has a size which corresponds to the surface of the damper (220) which is connected to the force introduction element and/or to the force transmission element.

5. A peritoneal dialysis machine in accordance with one of the preceding claims, **characterized in that** the damper (220) is cylindrical.

6. A peritoneal dialysis machine in accordance with one of the preceding claims, **characterized in that** the damper (220) comprises rubber or consists of rubber.

7. A peritoneal dialysis machine in accordance with one of the claims 2 to 6, **characterized in that** the holder (240) extends through at least one opening (300) of the housing of the weighing cell (200), with the opening being dimensioned such that the holder (240) does not contact the opening margin in at least one position of the drain pan (140).

8. A peritoneal dialysis machine in accordance with claim 7, **characterized in that** the named position is that position of the drain pan (140) in which it is located in its non-loaded position of rest, with provision preferably being made that the opening (300) has a diameter which is at least twice as large as the outer diameter of the holder (240).

9. A peritoneal dialysis machine in accordance with one of the preceding claims, **characterized in that** the weighing cell (200) is arranged in or at the machine housing.

10. A peritoneal dialysis machine in accordance with one of the preceding claims, **characterized in that** the drain pan (140) is located beneath the machine housing (120) and the weighing cell (200) is located in the machine housing (120).

11. A peritoneal dialysis machine in accordance with one of the preceding claims, **characterized in that** at least one, preferably adjustable, abutment (400) is located beneath the housing of the weighing cell (220) which limits the deflection of the housing of the weighing cell (200).

12. A peritoneal dialysis machine in accordance with claim 11, **characterized in that** the abutment is formed as a plate (400), in particular a metal plate, that extends beneath the installation plate (P) and that is spaced apart therefrom at least region-wise.

13. A peritoneal dialysis machine in accordance with claim 11 or claim 12, **characterized in that** adjustment means (420, 421)are provided by means of which the spacing between the housing of the weighing cell (200) and the abutment (400) is variable, with provision preferably being made that the adjustment means (420, 421) comprise at least one screw (420) and at least one locknut (421).

## Revendications

1. Appareil de dialyse péritonéale comprenant au moins un boîtier d'appareil (120) et comprenant au moins une cuvette de drainage (140) pour recevoir une ou plusieurs poches de solution destinées à conserver du dialysat usagé, la cuvette de drainage (140) étant en liaison avec au moins une cellule de pesée (200) de telle manière que le poids de la cuvette de drainage (140) peut être détecté au moyen de la cellule de pesée (200),
**caractérisé en ce que**
au moins un système d'amortissement est prévu pour empêcher l'occurrence de l'application d'une force non admissible dans la cellule de pesée (200), le système d'amortissement comportant au moins un amortisseur (220), sur lequel la cuvette de drainage (140) est fixée directement ou indirectement, l'amortisseur (220) étant en liaison avec la cellule de pesée (200) de telle manière que le poids de la cuvette de drainage (140) agissant sur celui-ci est transmis à la cellule de pesée (200), au moins un support (240) étant prévu, qui porte la cuvette de drainage (140) et qui est disposé directement ou indirectement sur l'amortisseur (220) et l'amortisseur (220) comportant au moins un passage, à travers lequel le support (240) s'étend et le dispositif d'amortissement comportant au moins un élément d'application de force sous la forme d'une plaque de recouvrement (210), qui se trouve au-dessus de l'amortisseur (220) et sur laquelle la cuvette de drainage (140) est fixée au moyen du support (240).

2. Appareil de dialyse péritonéale selon la revendication 1, **caractérisé en ce que** l'amortisseur (220) comporte un axe longitudinal et **en ce que** le passage s'étend le long de l'axe longitudinal ou le long d'un axe s'étendant parallèlement à celui-ci.

3. Appareil de dialyse péritonéale selon l'une des revendications précédentes, **caractérisé en ce que** le dispositif d'amortissement comporte au moins un élément de transmission de force, de préférence au moins une plaque de fond (230), qui est disposée entre l'amortisseur (220) et le boîtier de la cellule de pesée (200).

4. Appareil de dialyse péritonéale selon la revendication 1 ou 3, **caractérisé en ce que** l'élément d'application de force, de préférence la plaque de fond (230), et/ou l'élément de transmission de force, de préférence la plaque de recouvrement (210), présente une dimension qui correspond à la surface de l'amortisseur (220), qui est en liaison avec l'élément d'application de force et/ou avec l'élément de transmission de force.

5. Appareil de dialyse péritonéale selon l'une des revendications précédentes, **caractérisé en ce que** l'amortisseur (220) est réalisé cylindrique.

6. Appareil de dialyse péritonéale selon l'une des revendications précédentes, **caractérisé en ce que** l'amortisseur (220) est constitué de caoutchouc ou comporte du caoutchouc.

7. Appareil de dialyse péritonéale selon l'une des revendications 2 à 6, **caractérisé en ce que** le support (240) s'étend à travers au moins une ouverture (300) du boîtier de la cellule de pesée (200), l'ouverture étant dimensionnée de telle manière que le support (240) ne touche pas le bord d'ouverture dans au moins une position de la cuvette de drainage (140).

8. Appareil de dialyse péritonéale selon la revendication 7, **caractérisé en ce que** ladite position est la position de la cuvette de drainage (140) dans laquelle celle-ci se trouve lorsqu'elle est en position de repos non sollicitée, l'ouverture (300) étant de préférence prévue de façon à présenter un diamètre qui est au moins deux fois plus grand que le diamètre extérieur du support (240).

9. Appareil de dialyse péritonéale selon l'une des revendications précédentes, **caractérisé en ce que** la cellule de pesée (200) est disposée dans ou sur le boîtier d'appareil (120).

10. Appareil de dialyse péritonéale selon l'une des revendications précédentes, **caractérisé en ce que** la cuvette de drainage (140) se trouve en dessous du boîtier d'appareil (120) et la cellule de pesée (200) se trouve dans le boîtier d'appareil (120).

11. Appareil de dialyse péritonéale selon l'une des revendications précédentes, **caractérisé en ce qu'**au moins une butée (400) de préférence réglable se trouve en dessous du boîtier de la cellule de pesée (200), ladite butée limitant le déplacement du boîtier de la cellule de pesée (200).

12. Appareil de dialyse péritonéale selon la revendication 11, **caractérisé en ce que** la butée est réalisée sous la forme d'une plaque (400), en particulier d'une plaque métallique, qui s'étend en dessous de la plaque de montage (P) et qui est espacée de celle-ci au moins dans certaines zones.

13. Appareil de dialyse péritonéale selon la revendication 11 ou 12, **caractérisé en ce que** des moyens de réglage (420, 421) sont prévus, au moyen desquels la distance entre le boîtier de la cellule de pesée (200) et la butée (400) peut être modifiée, les moyens de réglage (420, 421) étant de préférence prévus de façon à comprendre au moins une vis (420) et au moins un écrou de blocage (421).
